# EUROPEAN PATENT SPECIFICATION

(11) **EP 2 057 974 B1**
(45) Date of publication and mention of the grant of the patent: **23.05.2012**
(21) Application number: 07806316.1
(22) Date of filing: 30.08.2007
(51) Int. Cl.: A61F 13/00, A61B 17/12, A61F 13/02

(54) **MEDICAL DEVICE FOR PRESSURIZING LIMBS AND TRUNK**
MEDIZINISCHE VORRICHTUNG ZUR UNTERDRUCKSETZUNG VON EXTREMITÄTEN UND RUMPF
DISPOSITIF MÉDICAL DESTINÉ À EXERCER UNE PRESSION SUR LES MEMBRES ET LE TRONC

(30) Priority: 30.08.2006 JP 2006234189
(43) Date of publication of application: 13.05.2009
(73) Proprietor: Doya, Hideo, Nishi-Tokyo-shi Tokyo 188-0001 (JP)
(72) Inventor: Doya, Hideo, Nishi-Tokyo-shi Tokyo 188-0001 (JP)
(74) Representative: Grünecker, Kinkeldey, Stockmair & Schwanhäusser
(86) International application number: PCT/JP2007/066839
(87) International publication number: WO 2008/026669

(56) References cited:
- DE-A1- 2 035 607
- DE-A1- 4 209 106
- DE-A1- 19 715 461
- FR-A- 1 263 391
- FR-A1- 2 806 904
- JP-A- 09 294 760
- JP-A- 10 114 357
- JP-A- 2002 513 634
- US-A- 4 991 234
- US-A- 5 015 251
- US-A- 5 468 219
- US-B1- 6 545 193

## Description

The present invention relates to a medical Instrument for applying compressing force to body trunk and extremities, and more particularly, it relates to a medical Instrument for treating and preventing edema in body trunk and extremities.

A medical instrument for the setting of broken extremities, wherein fastening strips impregnated with a curable resin are attached to a fiberglass bandage in such a way so that when the bandage is wrapped around the injured extremity said fastening strips overlap in a cross-mesh fashion to form a stable structure, is known from US 5 468 219 A.

### BACKGROUND OF THE INVENTION

One of edema related syndromes is lymphedema that causes lymph fluid to excessively build up in clearances of the body tissues or in other body cavities, and the lymphedema is disclosed in, for example, Non-Patent Document 1 listed below.

Lymphedema results from a disorder of lymph circulation, and especially, an irreversible type of lymphedema is prone to occur as a post-operation sequela after lymphadenectomy in malignant tumor resection surgery or radiation therapy, which is disclosed in, for example, Non-Patent Document 2 listed below.

A typical treatment for lymphedema is multilayer bandage technique, as disclosed in Non-Patent Document 3, where tissue pressure is altered to stimulate lymph drainage.

### [Non-Patent Document 1]

Rehabilitation Approach to Lymphedema, The Rehabilitation Medicine, vol. 43, No. 1 pp. 51-62 (2006), by Kanoko Hosokawa, Kazumi Kondoh, Manabu Iwata

### [Non-Patent Document 2]

Complex Decongestive Physical Threrapy for Secondary Lymphedema in Lower Extremities - Introduction of CPD, The Monthly Nurse Data, Vol. 26, No. 2, pp. 14-25 (2005), by Hirokazu Yoshihara

### [Non-Patent Document 3]

Lymphedema, pp. 153-86, (Chuohoki Shuppan 2003), by Robert Twycross, Karen Jenns, Jacqelyne Todd

The multilayer bandage technique is a therapeutic expertise that can be mastered by none but a dedicated person trained under the supervision and direction of a seasoned therapist (Non-Patent Document 3), and since the multilayer bandage technique requires skilled technique and adjustment for the conditions of edema, it is actually difficult for a patient to manage the edema 24 hours a day (Non-Patent Document 1).

Applying compression garments is easy to use without therapeutic expertise, but instead, troubles due to mal-fitting arise; that is, the wrapping that does not fit the patient may be too loose over the lesion for the desired compressing effect, or it may be so tight as it causes pain for the patient (Non-Patent Document 1).

### SUMMARY OF THE INVENTION

The present invention is made to overcome the aforementioned disadvantages and accordingly, it is an object of the present invention to provide a compression bandage that enables a person without therapeutic expertise to easily apply it in position without loosening, for an appropriate compressing force over the lesion.

In order to achieve the above-mentioned object, a medical instrument according to claim 1 is provided.

A medical instrument compressing force to body trunk and extremities according to the present invention comprises a bandage-like compression fabric and fastening means for releasably fastening opposed first and second surfaces of the compression fabric to each other at a plurality of points in a longitudinal direction of the compression fabric.

With such a medical instrument, as the compression fabric is shaped like a bandage, it is easy to wrap around an extremity of the human body while applying the desired compressing force. Additionally, since the first and second surfaces of the compression fabric can be fastened to each other during the wrapping, even if the user stops to hold the compression fabric, a portion(s) of the compression fabric once wrapped around the body is not disengaged from the body. Further, since the fastening means releasably fastens the first and second surfaces of the compression fabric to each other, it is easy to rewrap the medical instrument around the body.

The fastening means includes male and female hook-and-loop fasteners provided in the first and second surfaces, respectively. These simple structures allow releasable fastening opposed first and second surfaces of the compression fabric to each other.

One of the male and female hook-and-loop fasteners is provided so as to extend across a length of the compression fabric which is to be wrapped around the body, the other of the male and female hook-and-loop fasteners consists of those spaced from each other in a longitudinal direction of the compression fabric. This allows releasable fastening of male and female hook-and-loop fasteners at an optional position.

The present invention provides a medical instrument to achieve an appropriate compressing force easily.

### BRIEF DESCRIPTION OF THE DRAWINGS

Figure 1 is a lateral view of an edema-therapy instrument for a crus according to the present invention.
Figure 2 is a bottom view of the edema-therapy instrument in Fig. 1.
Figure 3 is a schematic view showing the edema-therapy instrument in a state that the instrument is being applied to the crus.
Figure 4 is a schematic view showing the edema-therapy instrument in a state that the instrument is being applied to the crus.
Figure 5 is a schematic view showing the edema-therapy instrument in a state that the instrument is being applied to the crus.

### DETAILED DESCRIPTION OF THE PREFERRED EMBODIMENTS

Preferred embodiments of the present invention will now be described with reference to the accompanying drawings. The embodiments will be discussed in connection with a therapeutic instrument for edema in lower extremities.

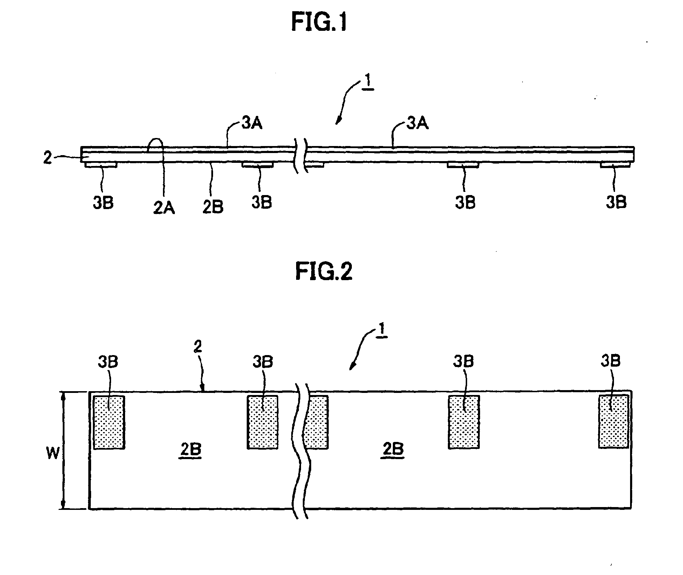

Referring to Fig. 1, a therapeutic instrument for edema in a crus (extending from the knee to the ankle) is generally indicated by a reference numeral 1.

The instrument 1 comprises a bandage-like compression fabric 2. The compression fabric 2 can be made of any of known materials sufficiently flexible to be wrapped around the crus, such as cotton, polyester, polyethylene, polyamide, polypropylene, viscose rayon, rubber, and the like.

The compression fabric 2 includes opposed first and second surfaces 2A and 2B and fastening means for releasably fastening the first and second surfaces 2A and 2B to each other. In this embodiment, the fastening means include a female (loop) hook-and-loop fastener 3A provided on the first surface 2A so as to extend across the entire length of the compression fabric 2, and a plurality of male (hook) hook-and-loop fasteners 3B provided on the second surface 2B so as to be equally spaced from each other across the entire length of the compression fabric 2.

Depending upon a body constitution of the patient and a site of the body to which the medical instrument 1 is applied, the plurality of the male (hook) fastener 3B may be unequally spaced from each other so that an optimum compressing force as mentioned below can be provided. Further, the hook-and-loop fasteners 3Aand 3B may be defined by a male (hook) fastener(s) and a female (loop) fastener, respectively.

As shown in Fig. 2, each of the male fasteners 3B may be disposed in a half width area of a full width (W) of the compression fabric 2.

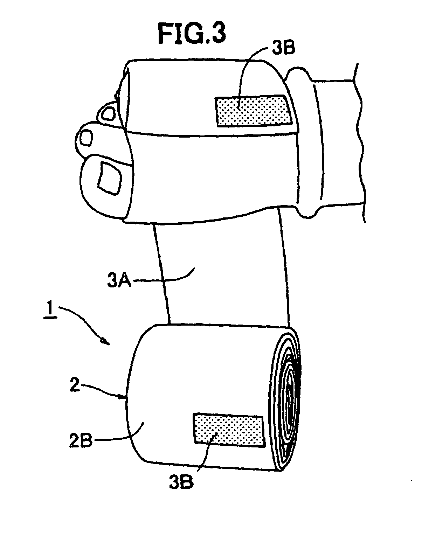

As an example, a procedure of wrapping the edema-therapy instrument 1 stated above around crus from its forefoot to knee will be described below. Further, it is convenient, before the edema-therapy instrument 1 is used, to wind up the same into a roll with the second surface 2B positioned outside as shown in Fig. 3.

Initially, a leading free end of the rolled (wound) edema-therapy instrument 1 is unwound from the rolled edema-therapy instrument 1 and then put on the patient's forefoot with the first surface 2A /the female (loop) fastener 3A coming into contact with the patient's forefoot.

After that, while the leading end of the edema-therapy instrument 1 is being held against the forefoot to avoid slipping off, the rolled edema-therapy instrument 1 is further unwound and then the unwound segment thereof is wrapped around the forefoot while being stretched with the desired force such that the first surface 2A /the female (loop) fastener 3A comes into contact with the patient's forefoot, thereby applying the desired compressing force to the forefoot.

The female (loop) fastener 3A of the first surface 2A of a further unwound segment of the edema-therapy instrument 1 is wrapped around the forefoot so as to overlie on the leading free end of the instrument 1 put on the forefoot, whereby the female (loop) fastener 3A of the first surface 2A of the further unwound segment is fastened to the male (hook) fastener 3B of the second surface 2B of the leading free end of the instrument 1 put on the forefoot. Thus, even if the user stops to hold the further unwound segment, the desired compressing force applied against the forefoot can be maintained. Therefore, the user can avoid an annoying task of rewrapping the edema-therapy instrument 1 around the forefoot due to the fact that the user fails to hold the instrument 1 against the forefoot to loose.

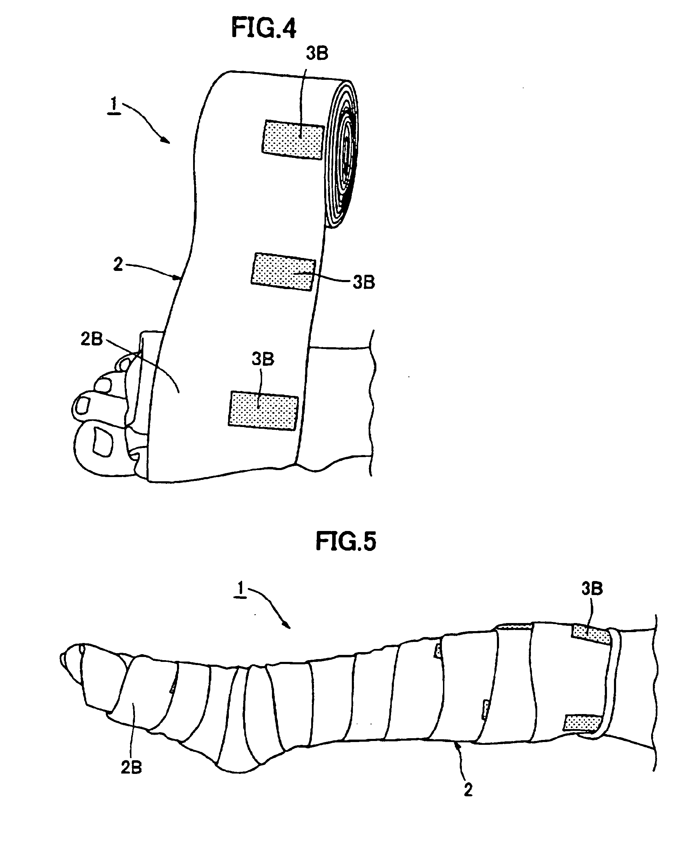

In order to wrap the edema-therapy instrument 1 around the crus from the forefoot up toward the knee, when a further unwound segment of the edema-therapy instrument 1 is wrapped over the externally exposed surface of the underlying winding (that is, when the female (loop) fastener 3A of the first surface 2A of the further unwound segment is fastened to the second surface 2B of the underlying winding), the wrapping portion (the unwound segment) should be offset from the wrapped portion (the underlying winding) in a proximal direction of the body, instead of the wrapping portion being perfectly aligned with the wrapped portion, as shown in Fig. 4. A greater overlapping area with the underlying layer results in the compression fabric 2 being fastened more securely around the human body, whereby the edema-therapy instrument 1 is more effectively prevented from being loosed and unwound.

Since it is sufficient for the male (hook) fastener 3B to fasten the female (loop) fastener 3A so as to maintain the desired compressing force against the human body, as far as the male (hook) fastener 3B satisfies this requirement, the male (hook) fastener 3B may be of any length relative to the full width of the compression fabric 2. In this connection, since the edema-therapy instrument 1 is being wrapped around the body with the wrapping portion offset from the wrapped portion in the proximal direction of the body, each of the male (hook) fasteners 3B do not need to extend across the full width W of the compression fabric 2. Thus, by forming each of the male (hook) fasteners 3B only in the half area of the full width (W) of the compression fabric 2 as in the present embodiment, the production of the edema-therapy instrument 1 can be facilitated and the manufacturing cost thereof can be reduced.

Because, in the present embodiment, each of the male (hook) fasteners 3B is disposed in the half area of the full width W of the compression fabric 2, when the edema-therapy instrument 1 is wrapped around the body, the edema-therapy instrument 1 is set relative to the body so that the male (hook) fasteners 3B are positioned proximal of the human body, as shown in Figs. 3 and 4.

The edema-therapy instrument 1 is further wrapped around the crus from the forefoot up to the knee in the aforementioned manner, as shown in Fig. 5. In wrapping the edema-therapy instrument I around the crus, an optimum compressing force can be provided for each individual patient, and in particular each individual region thereof. Additionally, during the wrapping procedure, portions of the edema-therapeutic instrument 1, in which the male (hook) and female (loop) fasteners 3A and 3B have been fastened to each other, would not be fortuitously released from the human body, and therefore, even if the user stops to hold the instrument 1, the user can avoid an annoying task of rewrapping the same around the body.

The present invention is not intended to limit the precise forms of the aforementioned embodiments, but it should be understood that the invention be modified in various ways within the scope of the claims.

The compression fabric 2 may be made of elastic material. According to this compression fabric 2, in the event of wrapping the instrument 1 around an region of the human body including a joint(s), a limitation to a movable range of the joint(s) can be reduced and the desired compressing force can be retained after the patient exercises the joint(s).

Further, although the aforementioned exemplary therapeutic instrument according to the invention has been described by way of example as edema-therapy instrument 1 for the patient's crus, the instrument can be used to apply a compressing force to various other regions of the human body, such as a femoral, a brachial, and a trunk, an amputated portion of limbs, and the like.

Furthermore, the therapeutic instrument according to the present invention can be used as a compression instrument for deep venous thrombosis and varicose veins.

## Claims

1. A medical instrument for applying compressing force to body trunk or extremities, comprising
a bandage-like compression fabric, and
fastening means for releasably fastening opposed first and second surfaces of the compression fabric to each other at a plurality of points in a longitudinal direction of the compression fabric,
said fastening means including male and female hook-and-loop fasteners provided in said first and second surfaces, respectively,
one of the male and female hook-and loop fasteners being provided so as to extend across a length of the compression fabric which is to be wrapped around the body trunk or extremity, the other of the male and female hook-and-loop fasteners consisting of those spaced from each other in a longitudinal direction of the compression fabric,
said compression fabric being adapted so that, when the medical instrument is wrapped around the body trunk or extremity from a first position to a second position thereof, one of the first and second surfaces of the compression fabric can be wrapped over the other thereof in a partially offset state from the other in a direction of the second position, wherein the male fastener or the male hook-and-loop fasteners is or are located in a half area of a full width of the compression fabric.

## Patentansprüche

1. Medizinisches Instrument zum Anwenden einer Druckkraft auf den Körperrumpf oder Extremitäten, aufweisend
ein bandagenartiges Druckgewebe und
eine Befestigungseinrichtung zum lösbaren Befestigen gegenüberliegender erster und zweiter Oberflächen des Druckgewebes miteinander an einer Mehrzahl von Punkten in einer Längsrichtung des Druckgewebes,
wobei die Befestigungseinrichtung Haken- und- Ösen- Befestiger enthält, jeweils vorgesehen in der ersten und zweiten Oberfläche, wobei
einer der Haken- und- Ösen- Befestiger so vorgesehen ist, um sich quer zu einer Länge des Druckgewebes zu erstrecken, das um den Körperrumpf oder Extremitäten geschlungen werden soll, während die anderen der Haken- und- Ösen- Befestiger aus denen bestehen, die in einer Längsrichtung des Druckgewebes beabstandet sind,
wobei das Druckgewebe so vorgesehen ist, dass dann, wenn das medizinische Instrument um den Körperrumpf oder die Extremitäten von einer ersten Position zu einer zweiten Position derselben geschlungen ist, dann eine der ersten oder zweiten Oberflächen des Druckgewebes über die andere derselben in einem teilweise versetzten Zustand von der anderen in einer Richtung der zweiten Position geschlungen werden, wobei der Haken oder die Haken- und- Ösen- Befestiger in einem halben Bereich einer gesamten Breite des Druckgewebes angeordnet ist oder sind.

## Revendications

1. Instrument médical pour appliquer une force de compression sur le tronc ou des extrémités du corps, comprenant :
un tissu de compression du type bandage, et
des moyens de fixation pour fixer l'une à l'autre, de manière amovible, des première et seconde surfaces opposées du tissu de compression, au niveau de plusieurs points dans un sens longitudinal du tissu de compression,
les moyens de fixation comprenant des fixations autoagrippantes avec une partie crochets et une partie velours prévues respectivement sur les première et seconde surfaces,
la partie crochets ou velours étant prévue de manière à s'étendre sur toute la longueur du tissu de compression qui doit être enroulée autour du tronc ou de l'extrémité du corps, tandis que la partie velours ou crochets se compose de fixations espacées dans un sens longitudinal du tissu de compression,
le tissu de compression étant adapté de telle sorte que lorsque l'instrument médical est enroulé autour du tronc ou de l'extrémité du corps d'un premier point à un second point de celui-ci ou de celle-ci, les première et seconde surfaces du tissu de compression puissent être enroulées l'une sur l'autre en étant en partie décalées l'une par rapport à l'autre en direction du second point, étant précisé que la ou les parties crochets de la fixation autoagrippante se trouvent sur une moitié de la largeur totale du tissu de compression.
